(19) Europäisches Patentamt
European Patent Office
Office européen des brevets

(11) EP 1 175 149 B1

(12) FASCICULE DE BREVET EUROPEEN

(45) Date de publication et mention de la délivrance du brevet: **11.12.2002 Bulletin 2002/50**

(21) Numéro de dépôt: **00922743.0**

(22) Date de dépôt: **25.04.2000**

(51) Int Cl.[7]: **A01N 59/16**
// (A01N59/16, 59:26, 59:16, 59:00, 43:40, 37:16, 25:22), A01N25:04

(86) Numéro de dépôt international: **PCT/FR00/01093**

(87) Numéro de publication internationale: **WO 01/005233 (25.01.2001 Gazette 2001/04)**

# (54) COMPOSITION DESINFECTANTE A BASE DE H2O2, ACIDES ET IONS METALLIQUES

DESINFIZIERENDE ZUSAMMENSETZUNG AUF BASIS VON H2O2, SÄUREN UND METALLIONEN

DISINFECTING COMPOSITION BASED ON H2O2, ACIDS AND METAL IONS

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **23.04.1999 FR 9905186**

(43) Date de publication de la demande:
**30.01.2002 Bulletin 2002/05**

(73) Titulaire: **Digol International Ltd.**
**Tortola (VG)**

(72) Inventeurs:
- **WILMOTTE, Rémi**
  **F-51140 Chalons-sur-Vesles (FR)**
- **LEBEAU, Bernard**
  **F-92700 Colombes (FR)**
- **IRURZUN, Jean-Pierre**
  **F-94420 Le Plessis Trevise (FR)**
- **MARECHAL, Françoise**
  **F-69001 Lyon (FR)**

(74) Mandataire: **Clisci, Serge et al**
**S.A. FEDIT-LORIOT & AUTRES**
**38, avenue Hoche**
**75008 Paris (FR)**

(56) Documents cités:
**EP-A- 0 233 059**
**WO-A-94/24863**
**WO-A-96/18301**
**DE-A- 2 659 462**
**GB-A- 2 189 394**
**US-A- 3 702 298**
**US-A- 3 907 991**
**US-A- 4 980 152**
**US-A- 4 995 987**
**US-A- 5 017 295**
**US-A- 5 078 902**
**US-A- 5 233 149**

- **PATENT ABSTRACTS OF JAPAN vol. 1997, no. 12, 25 décembre 1997 (1997-12-25) & JP 09 202983 A (KAMIUSUKI TOSHIAKA), 5 août 1997 (1997-08-05)**
- **DATABASE WPI Section Ch, Derwent Publications Ltd., London, GB; Class B05, AN 1972-29295T XP002141990 & JP 47 014756 B (TANABE SEIYAKU CO)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 175 149 B1

## Description

### *Domaine de l'invention*

La présente invention concerne en tant que produit industriel nouveau une composition aqueuse, notamment sous forme de gel, à base de $H_2O_2$, d'acides et d'ions de $Ag^{II}$, $Ag^{III}$, $V^{V}$, $Nb^{V}$, $Ta^{V}$, $Mo^{VI}$, $W^{VI}$, $Co^{III}$, $In^{III}$ ou $Tl^{III}$. Elle concerne également un procédé de préparation, notamment quand lesdits ions sont $Ag^{2+}$, et l'utilisation de cette composition dans le domaine de la désinfection, de l'hygiène et/ou de la dépollution, d'une part, et dans le domaine du traitement de surface (en particulier le nettoyage, le décapage et/ou la passivation), d'autre part.

### *Art antérieur*

L'art antérieur le plus proche est constitué par le document de brevet WO-A-96/18301. Avant la publication de ce document, on avait déjà proposé dans le passé des compositions aqueuses désinfectantes contenant, les unes du peroxyde d'hydrogène et un mélange acide carboxylique/peracide carboxylique du type $RCO_2H/RCO_3H$ (où R est essentiellement $CH_3$ ou $CH_3CH_2$) (voir en particulier à cet effet EP-A-0370850, EP-A-0193416, EP-B-0087343, FR-A-2321301 et FR-A-2321302), et les autres du peroxyde d'hydrogène et des ions $Ag^+$ (voir notamment à cet effet US-A-3035698 et GB-A-2189394), lesdites compositions aqueuses désinfectantes étant stabilisées dans chaque cas par un acide fort (essentiellement $H_3PO_4$).

Ces compositions aqueuses désinfectantes antérieures, à savoir (i) celles du type $H_2O_2 + RCO_2H/RCO_3H + H_3PO_4$ et (ii) celles du type $H_2O_2 + Ag^+ + H_3PO_4$, sont insuffisantes eu égard à leur absence d'efficacité sur plusieurs souches de bactéries et de moisissures, et en particulier sur les souches de ***Penicillium verrucosum.***

Une technique plus récente et nettement plus efficace (notamment vis-à-vis desdites souches de ***Pénicillium verrucosum*** qui étaient résistantes aux deux types sus-visés de compositions aqueuses désinfectantes) a été décrite dans WO-A-96/18301 précité, qui est incorporé ici à titre de référence. Selon WO-A-96/18301, la technique préconisée fait appel à une composition aqueuse désinfectante, d'hygiène et dépolluante, qui comprend dans de l'eau :

(A) $H_2O_2$,
(B) un mélange $RCO_2H/RCO_3H$ (où R est $CH_3$ ou $CH_3CH_2$),
(C) un composant d'argent en tant que source d'ions $Ag^+$, et
(D) un agent stabilisant (principalement $H_3PO_4$),

le tableau IX de WO-A-96/18301 mettant en évidence la synergie de la composition aqueuse contenant les composants A+B+C+D, par rapport aux compositions aqueuses contenant les composants A+B+D et A+C+D.

Il se trouve que la solution aqueuse de A+B+C+D selon WO-A-96/18301 n'agit pas assez rapidement quand il s'agit d'inhiber ou mieux de détruire les microorganismes, tels que les bactéries, les moisissures, les virus et les algues microscopiques, en particulier en fragilisant leur paroi, notamment par dégradation de leur biofilm, en perforant leur paroi ou encore en empêchant leur pénétration dans les cellules de l'organisme ou en protégeant lesdites cellules de leurs toxines.

Par ailleurs, les modalités opératoires décrites dans GB-A-2189394 en ce qui concerne la préparation de la solution aqueuse concentrée en argent (i.e. le composant C ci-dessus) en présence de $H_3PO_4$, d'une part, et celle de la solution aqueuse résultante concentrée en argent et en peroxyde d'hydrogène et stabilisée (i.e. la solution aqueuse contenant A+C+D), d'autre part, ne conduisent pas à la production d'ions $Ag^{2+}$ ni à celle d'ions $Ag^{3+}$.

En effet GB-A-2189394, qui préconise un procédé comprenant :

- le mélange d'un acide minéral fort (pH<1,6) avec un composant argent (un sel d'argent ou un complexe d'argent), à une température de 50-60°C, le rapport molaire acide minéral fort/composant argent étant supérieur ou égal à 1/1 ;
- le refroidissement du mélange résultant à une température de 25-30°C et l'ajout d'un acide organique stabilisant avec éventuellement de la gélatine ; et,
- l'incorporation de $H_2O_2$ dans le mélange résultant,

cite (voir page 1 lignes 46-50) des composés d'argent (I) qui fournissent en milieu acide fort des ions $Ag^+$, il s'agit de sels d'argent (I) et d'un sel complexe d'argent (I) à savoir $AgNaCl_2$, d'une part, et des composés d'argent (II) et (III) qui ne sont solubles qu'en milieu basique, il s'agit d'oxydes d'argent (II) et (III) qui en milieu acide fort donnent principalement de l'argent métallique *in fine,* d'autre part.

Plus précisément, les oxydes d'argent (I), (II) et (III) donnent lieu, en milieu acide, aux réactions suivantes, où s désigne un produit solide :

(1) $Ag_2O(s) + 2H^+ + 2e \rightarrow 2Ag(s) + H_2O$

(2) $2AgO(s) + 2H^+ + 2e \rightarrow Ag_2O(s) + H_2O$

(3) $Ag_2O_3(s) + 2H^+ + 2e \rightarrow 2AgO(s) + H_2O$

Comme la mise en contact direct (i.e. sans stabilisant acide fort) de $H_2O_2$ et d'un composant d'argent est susceptible de provoquer une explosion, il faut mettre en oeuvre un procédé différent de celui préconisé par GB-A-2189394 pour obtenir les ions $Ag^{2+}$ utiles selon la présente invention.

Pour les mêmes raisons, les modalités opératoires décrites dans WO-A-96/18301 ne permettent pas l'oxydation en milieu acide de l'argent (I) en argent (II) selon la réaction : $Ag^+ \rightarrow Ag^{2+} + e$.

On sait que, selon US-A-5017295 et US-A-5078902, les ions $Ag^{2+}$ ont été présentés comme étant plus actifs en tant qu'agents bactéricides que les ions $Ag^+$. Or il se trouve que les modalités opératoires fournies dans ces deux documents ne favorisent pas l'obtention d'ions $Ag^{2+}$ ou $Ag^{2+} + Ag^{3+}$ car eu égard à la réaction (2) ci-dessus l'oxyde d'argent (II) donne en milieu acide un précipité d'oxyde d'argent (I). Ainsi, les réactions visées à l'exemple 1 de US-A-5017295 et aux exemples 1 et 2 de US-A-5078902, à savoir :

$$3AgO + 2H_3PO_4 \rightarrow Ag_3(PO_4)_2 + 3H_2O,$$

$$AgO + 2HNO_3 \rightarrow Ag(NO_3)_2 + H_2O,$$

et

$$AgO + 2HCl \rightarrow AgCl_2 + H_2O,$$

ne peuvent avoir lieu. De plus en reproduisant l'autre mode d'obtention d'ions $Ag^{2+}$ indiqué à l'exemple 2 de US-A-5078902, qui comprend la dissolution de AgO en milieu alcalin puis l'addition d'une source d'oxydant [i.e. oxone ($Na_2O_2$)] pouvant donner du peroxyde d'hydrogène [cette addition est faite en milieu alcalin (i.e. dans des conditions où $H_2O_2$ intervient en tant qu'agent réducteur)], la concentration résultante en ions $Ag^{2+}$ est très faible de sorte que le potentiel d'oxydo-réduction de la solution aqueuse contenant $Ag^+$ et $Ag^{2+}$ est toujours inférieure à 650 mV (à 298 K). Ainsi selon US-A-5017295 et US-A-5078902 les effets bénéfiques tels qu'annoncés des ions $Ag^{2+}$ ne peuvent pas se manifester.

Enfin US-A-3702298 propose l'emploi d'ions $Ag^{3+}$ en tant que. moyen germicide. Cependant le mélange $Ag^+ + Ag^{2+} + Ag^{3+}$ effectivement obtenu possède un potentiel d'oxydo-réduction allant de 0,15 mV à 0,4 mV, qui est nettement inférieur à celui recherché selon la présente invention qui est supérieur à 1 000 mV.

On sait aussi que le molybdène intervient chez l'homme et les animaux à sang chaud en tant que co-enzyme des enzymes détoxifiants localisés au niveau hépatique, tels que les xanthine oxydase, aldéhyde oxydase et sulfite oxydase. Mo est essentiel pour la transformation des bases puriques en acide urique, il favorise l'absorption intestinale du fer et la rétention du fluor dans l'organisme.

On sait en outre de US-A-4995987 que les ions molybdate et tungstate ont été proposés, en tant qu'agents inhibant la croissance des bactéries réduisant les sulfates, en association avec des agents antimicrobiens. Le document US-A-4 995 987 ne décrit ni ne suggère l'objet de la présente invention.

Dans le domaine du traitement des surfaces métalliques notamment pour les décaper et/ou les passiver, on sait que les moyens classiques oxydants, tels que l'acide chromique, les chromates et l'acide nitrique, sont considérés comme étant des produits nocifs pour l'environnement.

On connaît par ailleurs de US-A-4537778 une composition aqueuse pour bain de bouche ou rinçage buccal qui comprend 0,5-5 % en poids de $H_2O_2$, 3-15 % en poids d'éthanol, 0,5-2 % en poids d'un agent tensioactif non-ionique et hydrosoluble du type polyoxypropylèneglycol polyoxyéthyléné, 0,3-2 % en poids d'un agent tensioactif non-ionique et hydrosoluble du type monoester d'acide gras en $C_{10}$-$C_{18}$ avec du sorbitol polyoxyéthyléné, un édulcorant et un parfum. Cette composition peut être amenée sous la forme d'une pâte ou d'un gel au moyen d'un épaississant ou respectivement d'un agent gélifiant. L'agent gélifiant proposé est une gomme (gomme xanthane, gomme guar), un interpolymère carboxylique décrit dans US-A-2798053 ou un polyol du type PLURONIC® comportant 70 à 20 % de

groupes hydrophobes et 30 à 80 % de groupes hydrophiles, l'agent gélifiant préféré étant le PLURONIC® 127 (voir colonne 4, lignes 28-29, colonne 5 lignes 34 et 49, et colonne 6 lignes 5-6). Or il se trouve qu'un tel agent gélifiant, qui est du type polymère organique, est dégradé plus ou moins rapidement (au bout de quelques minutes à quelques jours) par les compositions oxydantes ayant un potentiel rédox élevé (notamment un potentiel rédox supérieur ou égal à 800 mV).

On connaît enfin de DE-A-2659462 une composition pharmaceutique contenant un gel de silice particulier, à savoir un hydrogel d'acide silicique qui intervient de façon usuelle dans l'art galénique mais n'a jamais été utilisé dans des conditions très oxydantes.

Enfin, l'art antérieur ne décrit ni ne suggère l'utilisation efficace d'ions contenant un métal à un degré d'oxydation voisin (cas particulier de $Ag^{II}$) ou égal (cas de $Ag^{III}$, $V^{V}$, $Nb^{V}$, $Ta^{V}$, $Mo^{VI}$, $W^{VI}$, $Co^{III}$, $In^{III}$ et $Tl^{III}$) à sa valeur maximale, dans une composition désinfectante ou de traitement de surface.

### *But de l'invention*

On veut pallier aux inconvénients précités de l'art antérieur, d'une part, et fournir une nouvelle solution technique efficace et inattendue par rapport à l'enseignement de l'art antérieur, d'autre part.

Selon un premier aspect de l'invention, l'on se propose de fournir une nouvelle solution technique au problème de la désinfection et/ou du traitement de surface, qui soit plus efficace en ce qui concerne la rapidité d'action que la solution technique préconisée par WO-A-96/18301.

Selon un second aspect, l'on se propose de fournir une nouvelle solution technique, qui présente une durée d'action allongée en vue d'obtenir une rémanence de l'activité au cours d'une période de temps plus longue, notamment quand la surface à traiter est inclinée ou verticale.

### *Objet de l'invention*

La nouvelle solution technique selon l'invention pour aboutir au but précité est fondée sur l'utilisation d'ions métalliques particuliers, à savoir des ions de $Ag^{II}$, $Ag^{III}$, $V^{V}$, $Nb^{V}$, $Ta^{V}$, $Mo^{VI}$, $W^{VI}$, $Co^{III}$, $In^{III}$ ou $Tl^{III}$.

On préconise ainsi une composition aqueuse oxydante (I), qui est utile notamment dans le domaine de la désinfection, de l'hygiène et de la dépollution, d'une part, et dans le domaine du traitement d'une surface métallique (en particulier pour son nettoyage, son décapage et/ou sa passivation) ou non métallique (notamment une surface en matière plastique ou en céramique, notamment pour son nettoyage et/ou son décapage), d'autre part, ladite composition, qui contient dans de l'eau du peroxyde d'hydrogène, un mélange $RCO_2H/RCO_3H$ (où R est un reste aliphatique en $C_1$-$C_6$ à chaîne hydrocarbonée linéaire ou ramifiée, saturée ou insaturée), et un agent stabilisant qui est un acide fort, et, le cas échéant, des ions $Ag^+$, étant caractérisée en ce qu'elle renferme des ions d'au moins un métal M qui sont des ions de $Ag^{II}$, $Ag^{III}$, $V^{V}$, $Nb^{V}$, $Ta^{V}$, $Mo^{VI}$, $W^{VI}$, $Co^{III}$, $In^{III}$ ou $Tl^{III}$, la source L, qui fournit des ions de $Ag^{II}$ ou des ions de $Ag^{II}$ + $Ag^{III}$, étant telle que, à partir de 100 g d'argent (i.e. 157 g de $AgNO_3$) et 1 litre d'eau déminéralisée, la concentration en ions $Ag^+ + Ag^{2+}$ ou $Ag^+ + Ag^{2+} + Ag^{3+}$ dans la solution résultante donne un potentiel d'oxydo-réduction supérieur à 1 000 mV.

Plus précisément, la composition (I) selon l'invention est caractérisée en ce qu'elle renferme dans de l'eau :

(A) $H_2O_2$ ;
(B) un mélange $RCO_2H/RCO_3H$ (où R est un reste aliphatique en $C_1$-$C_6$ à chaîne hydrocarbonée linéaire ou ramifiée, saturée ou insaturée ;
(C) des ions métalliques provenant d'une source L et choisis parmi l'ensemble constitué par :

  (1) les ions d'un métal M qui sont des ions de $Ag^{II}$, $Ag^{III}$, $V^{V}$, $Nb^{V}$, $Ta^{V}$, $Mo^{VI}$, $W^{VI}$, $Co^{III}$, $In^{III}$ ou $Tl^{III}$,
  (2) les associations d'ions $Ag^+$ et d'ions dudit métal M, et
  (3) leurs mélanges ;
  la source L, qui fournit des ions de $Ag^{II}$ ou des ions de $Ag^{II}$ + $Ag^{III}$, étant telle que, à partir de 100 g d'argent (i.e. 157 g de $AgNO_3$) et 1 litre d'eau déminéralisée, la concentration en ions $Ag^+ + Ag^{2+}$ ou $Ag^+ + Ag^{2+} + Ag^{3+}$ dans la solution résultante donne un potentiel d'oxydo-réduction supérieur à 1 000 mV ; et,

(D) un agent stabilisant qui est un acide.

Cette composition peut être préparée selon une méthode connue en soi. Le procédé que l'on préconise ici comprend les étapes consistant à :

(a) faire appel à une solution aqueuse d'une source L fournissant les ions métalliques choisis parmi l'ensemble

constitué par

(1) les ions d'un métal M qui sont des ions de $Ag^{II}$, $Ag^{III}$, $V^{V}$, $Nb^{V}$, $Ta^{V}$, $Mo^{VI}$, $W^{VI}$, $Co^{III}$, $In^{III}$ ou $Tl^{III}$,
(2) les associations d'ions $Ag^+$ et d'ions dudit métal M, et
(3) leurs mélanges ;

la source L, qui fournit des ions de $Ag^{II}$ ou des ions de $Ag^{II}$ + $Ag^{III}$, étant telle que, à partir de 100 g d'argent (i.e. 157 g de $AgNO_3$) et 1 litre d'eau déminéralisée, la concentration en ions $Ag^+ + Ag^{2+}$ ou $Ag^+ + Ag^{2+} + Ag^{3+}$ dans la solution résultante donne un potentiel d'oxydo-réduction supérieur à 1 000 mV ;
(b) introduire dans ladite solution l'agent stabilisant ;
(c) introduire ladite solution résultante, ainsi obtenue, dans de l'eau oxygénée ou introduire l'eau oxygénée dans ladite solution résultante ;
(d) introduire dans la solution résultante, ainsi obtenue, une substance acide qui est $RCO_3H$, $RCO_2H$ ou leur mélange $RCO_3H + RCO_2H$ où R est défini comme indiqué ci-dessus ;
(e) laisser reposer la solution résultante, ainsi obtenue, jusqu'à ce que l'équilibre $H_2O_2 + RCO_2H \Leftrightarrow H_2O + RCO_3H$ soit établi ; et,
(f) compléter jusqu'à 100 % en poids avec de l'eau.

Le procédé de préparation d'une composition selon l'invention qui contient des ions $Ag^{2+}$ et le cas échéant des ions $Ag^{3+}$ est caractérisé en ce qu'il comprend les étapes consistant à :

(1°) préparer une solution aqueuse d'un composant d'argent, qui intervient en tant que source d'ions $Ag^+$ ;
(2°) oxyder au moins une partie des ions $Ag^+$ en ions $Ag^{2+}$ au moyen de persulfate, de préférence avec du persulfate de sodium $Na_2S_2O_8$ ou du persulfate d'ammonium $(NH_4)_2S_2O_8$, de façon à obtenir un potentiel rédox supérieur ou égal à 1000 mV à partir de 1 litre d'eau déminéralisée et de 157 g de $AgNO_3$, et filtrer pour écarter l'insoluble susceptible d'être présent ;
(3°) introduire dans la solution résultante, ainsi obtenue, l'agent stabilisant ;
(4°) introduire ladite solution résultante, ainsi obtenue, dans de l'eau oxygénée ou introduire l'eau oxygénée dans ladite solution résultante ;
(5°) introduire dans la solution résultante, ainsi obtenue, une substance acide qui est $RCO_3H$, $RCO_2H$ ou leur mélange $RCO_3H + RCO_2H$ où R est défini comme indiqué ci-dessus ;
(6°) laisser reposer la solution résultante, ainsi obtenue, jusqu'à ce que l'équilibre $H_2O_2 + RCO_2H \Leftrightarrow H_2O + RCO_3H$ soit établi ; et,
(7°) compléter jusqu'à 100 % en poids avec de l'eau.

Lors de la mise en oeuvre de ce procédé des ions $Ag^{3+}$ sont susceptibles d'être obtenus au moins à l'état de traces.
Selon l'invention l'on préconise également une composition gélifiée (II), qui est caractérisée en ce qu'elle comprend :

- la composition aqueuse (I) sus-visée, et
- un agent gélifiant.

En pratique, la composition gélifiée (II) comportera 50 à 99,7 % en poids de ladite composition aqueuse (I) et 50 à 0,3 % en poids d'agent gélifiant. Plus précisément l'agent gélifiant préféré sera une silice colloïdale pyrogénée ou un composé du type polyacrylique tel que le CARBOPOL® ETD 2623 de la société GOODRICH, qui est un copolymère d'acrylate d'alkyle en $C_{10}$-$C_{30}$, ou le CARBOPOL® 672 également de la société GOODRICH, qui est un homopolymère polyacrylique.
Le procédé de préparation de la composition gélifiée (II) consiste (i) à mettre en contact ladite composition (I) avec l'agent gélifiant, (ii) à agiter le mélange résultant pendant 3 à 20 minutes, et (iii) à laisser reposer pour prise en masse du mélange résultant.

***Description détaillée de l'invention***

Dans ce qui suit, sauf indications contraires, les quantités respectives des ingrédients de la composition aqueuse décontaminante selon l'invention sont exprimées en % en poids, et les dilutions de ladite composition sont exprimées selon le rapport volume initial/volume de la composition diluée résultante.

- Le peroxyde d'hydrogène

D'une manière générale la composition aqueuse (I) selon l'invention contient une teneur en $H_2O_2$ qui est inférieure ou égale à 60 % en poids par rapport au poids de ladite composition. Ainsi la composition (I) selon l'invention peut contenir 0,1 à 60 % en poids de $H_2O_2$ et est susceptible d'être diluée au moment de l'utilisation (quand la concentration en $H_2O_2$ est supérieure ou égale à notamment 4 % en poids.

En pratique comme le peroxyde d'hydrogène soulève des difficultés sur le plan du transport, quand on fait appel à de l'eau oxygénée ayant une teneur en $H_2O_2$ supérieure à 8 % en poids, eu égard notamment aux règlements français et communautaires, on a intérêt à fournir une composition aqueuse (I) contenant au plus 8 % en poids et mieux au plus 7,9 % en poids de $H_2O_2$.

Par suite, la composition aqueuse (I) selon l'invention comprendra avantageusement une teneur en $H_2O_2$ de l'ordre de 7,5-7,9 % en poids et sera diluée, au moment de l'emploi, avec de l'eau jusqu'à notamment une concentration finale en $H_2O_2$ inférieure ou égale à 4 % en poids.

- Mélange $RCO_2H/RCO_3H$

Eu égard à la réaction d'équilibre (4) :

$$(4) \qquad H_2O_2 + RCO_2H \Leftrightarrow H_2O + RCO_3H$$

les quantités respectives de $RCO_3H$ et $RCO_2H$ dans le mélange $RCO_3H/RCO_2H$ ne sont pas critiques. Il suffit d'avoir en contact dans $H_2O$ soit $H_2O_2$ et $RCO_3H$ soit $H_2O_2$ et $RCO_2H$ pour obtenir un mélange ternaire $H_2O_2$ + $RCO_3H$ + $RCO_2H$ dès lors que $H_2O_2$ est en excès par rapport au couple $RCO_2H$/ $RCO_3H$. Aussi il suffit en quelque sorte d'incorporer :

(i) $RCO_2H$ en présence de $H_2O_2$, ou
(ii) $RCO_3H$ (qui à l'état concentré contient généralement $H_2O_2$ et $RCO_2H$ selon les documents FR-A-2 321 301 et FR-A-2 321 302 précités),

dans $H_2O$, pour obtenir à l'équilibre l'ensemble $H_2O_2$ + $RCO_3H$ + $RCO_2H$.

Comme indiqué plus haut le groupe R du couple acide/peracide représente un reste aliphatique en $C_1$-$C_6$ à chaîne hydrocarbonée linéaire ou ramifiée, saturée ou insaturée. De façon avantageuse, on fera appel à un groupe R à chaîne hydrocarbonée linéaire saturée telle que $CH_3$, $CH_3CH_2$ ou $CH_3(CH_2)_4$ ou bien à un groupe R à chaîne hydrocarbonée linéaire insaturée telle que notamment $CH_3$-CH=CH, $CH_3$-CH=CH-$CH_2$ ou $CH_3$-CH=CH-CH=CH.

Les groupes R préférés sont (selon un ordre de préférence croissant) : $CH_3$-CH=CH-CH=CH, $CH_3CH_2$ ou $CH_3$. D'une manière générale, on préfère le couple

$$CH_3CO_2H/CH_3CO_3H \text{ (i.e. R = méthyle)}$$

au couple

$$CH_3CH_2CO_2H/CH_3CH_2CO_3H \text{ (i.e. R = éthyle)}$$

dès lors que le premier couple est plus actif que le second en tant que moyen désinfectant/dépolluant dans la composition aqueuse selon l'invention.

De façon avantageuse, dans la composition aqueuse (I) selon l'invention, le rapport pondéral B/A du mélange $RCO_2H/RCO_3H$ au peroxyde d'hydrogène sera compris entre 0,15/1 et 0,85/1. De préférence, ce rapport pondéral sera compris entre 0,5/1 et 0,7/1.

- La source L

La source L a pour objet de fournir les ions utiles selon l'invention, à savoir les ions contenant le métal M (i. e. les ions de $Ag^{II}$, $Ag^{III}$, $V^V$, $Nb^V$, $Ta^V$, $Mo^{VI}$, $W^{VI}$, $Co^{III}$, $In^{III}$ où $Tl^{III}$), l'association $Ag^+$/ions du métal M, ou leurs mélanges.

Quand on veut des ions de $V^V$, $Nb^V$, $Ta^V$, $Mo^{VI}$, $W^{VI}$, $Co^{III}$, $In^{III}$ ou $Tl^{III}$, on fera appel à une source L qui est un sel dans lequel ledit métal M est à son degré d'oxydation maximal. En variante, l'on pourra utiliser comme source

L un oxyde dudit métal M (ici un oxyde de $V^V$, $Nb^V$, $Ta^V$, $Mo^{VI}$, $W^{VI}$, $Co^{III}$, $In^{III}$ ou $Tl^{III}$) si ledit oxyde est soluble dans les acides.

Quand on veut des ions $Ag^{2+}$, il convient de procéder à l'oxydation de $Ag^+$ (provenant avantageusement de $AgNO_3$ ou de l'argent colloïdal) en $Ag^{2+}$.

Les modalités opératoires préconisées pour la réaction $Ag^+ \rightarrow Ag^{2+} + e$ sont données plus loin. Cette réaction étant généralement incomplète, elle fournit principalement un mélange d'ions $Ag^+/Ag^{2+}$ [et, le cas échéant, un mélange d'ions $Ag^+/Ag^{2+}/Ag^{3+}$ où les ions $Ag^{3+}$ sont au moins à l'état de traces].

Quand on veut des ions $Ag^+$ en association avec des ions de $V^V$, $Nb^V$, $Ta^V$, $Mo^{VI}$, $W^{VI}$, $Co^{III}$, $In^{III}$ ou $Tl^{III}$, on mélange les ions provenant des deux sources correspondantes.

Enfin quand on veut un mélange ionique $Ag^+/Ag^{2+}$/ions de $V^V$, $Nb^V$, $Ta^V$, $Mo^{VI}$, $W^{VI}$, $Co^{III}$, $In^{III}$ ou $Tl^{III}$, on mélange les ions $Ag^+/Ag^{2+}$, obtenus à partir de leur source par oxydation comme indiqué ci-dessus, avec les ions de la source sus-visée d'ions $V^V$, $Nb^V$, $Ta^V$, $Mo^{VI}$, $W^{VI}$, $Co^{III}$, $In^{III}$ ou $Tl^{III}$.

Les ions préférés selon l'invention comprennent les ions de $V^V$, $Mo^{VI}$, $W^{VI}$ et $Tl^{III}$, d'une part, et les mélanges ioniques $Ag^+/Ag^{2+}$, $Ag^+/Mo^{VI}$, $Ag^+/W^{VI}$, $Ag^+/Tl^{III}$ et $Ag^+/Ag^{2+}/Mo^{VI}$, d'autre part, les ions les plus intéressants étant $Ag^+/Ag^{2+}$, $Ag^+/Mo^{VI}$, $Ag^+/Ag^{2+}/Mo^{VI}$ et surtout les ions de $Mo^{VI}$.

De façon inattendue, il a été observé, lors de la mise en oeuvre de la composition aqueuse (I) selon l'invention, que le molybdène présente un double intérêt vis-à-vis de l'argent (sous forme $Ag^+$ ou $Ag^+/Ag^{2+}$) :

- vis-à-vis des microorganismes, il est plus actif que l'argent, et
- lors de la stérilisation à froid d'instruments (dentaires ou chirurgicaux notamment), il ne donne pas les dépôts inesthétiques gris ou noirs de l'argent.

La principale source d'ions de $V^V$ est un sel de $VO_2^+$ (ou le cas échéant de $VO_3^-$), celle d'ions de $Nb^V$ un sel de $NbO^{3+}$, celle d'ions de $Mo^{VI}$ un sel de $MoO_2^{2+}$ ou de $MoO_4^{2-}$, celle d'ions de $Co^{III}$ un sel de $Co^{3+}$, celle d'ions de $W^{VI}$ un sel de $WO_4^{-2}$, celle d'ions de $Ta^V$ un sel de $Ta^{5+}$, celle d'ions de $In^{III}$ un sel de $In^{3+}$, et celle d'ions de $Tl^{III}$ un sel de $Tl^{3+}$.

Les ions du composant C selon l'invention agissent sur les microorganismes en fragilisant ou en dégradant leur paroi, d'une part, ils interviennent également en tant que moyens oxydants notamment selon les réactions suivantes, d'autre part.

$$(5) \qquad VO_2^+ + 2H^+ + e \rightarrow VO^{2+} + H_2O$$

ou, le cas échéant,

$$(5a) \qquad VO_3^- + 4H^+ + e \rightarrow VO^{2+} + 2H_2O$$

$$(6) \qquad Nb^V + 2e \rightarrow Nb^{III}$$

ou

$$(6a) \qquad NbO^{3+} + 2H^+ + 2e \rightarrow Nb^{3+} + H_2O$$

$$(7) \qquad MoO_2^{2+} + 2H^+ + e \rightarrow MoO^{3+} + H_2O$$

ou

$$(7a) \qquad MoO_4^{2-} + 4H^+ + e \rightarrow MoO_2^+ + 2H_2O$$

$$(8) \qquad Co^{3+} + e \rightarrow Co^{2+}$$

(en milieu acide)

(9) $In^{3+} + 2e \rightarrow In^{+}$

(10) $Tl^{3+} + 2e \rightarrow Tl^{+}$.

D'une façon pratique, il est recommandé sur le plan de l'efficacité que la source L puisse fournir, à partir d'une quantité correspondant à 100 g de métal M et 1 litre d'eau déminéralisée, une solution résultante contenant des ions de $Ag^{II}$, $Ag^{III}$, $V^{V}$, $Nb^{V}$, $Ta^{V}$, $Mo^{VI}$, $W^{VI}$, $Co^{III}$, $In^{III}$ et/ou $Tl^{III}$ ayant un potentiel d'oxydo-réduction supérieur à 1V.
De façon avantageuse, le rapport pondéral de la source L d'ions au peroxyde d'hydrogène sera compris entre 0,0005/1 et 0,015/1. De façon pratique, ce rapport pondéral sera encore plus avantageusement compris entre 0,0008/1 et 0,005/1 et mieux de l'ordre de 0,001/1.

- L'agent stabilisant

L'agent stabilisant, qui protège $H_2O_2$ et les ions de la source L et qui évite tout risque d'explosion, notamment à partir de solutions concentrées en (i) $H_2O_2$ et en (ii) $Ag^+$ et/ou $Ag^{2+}$, est choisi parmi l'ensemble constitué par les acides minéraux tels que $H_3PO_4$ et $H_2SO_4$ (les acides nitriques et chlorhydriques étant fortement déconseillés en raison, en particulier, de leurs effets nocifs sur les surfaces métalliques et/ou l'environnement), d'une part, et les acides organiques tels que les acides pyridinecarboxyliques, d'autre part. Le stabilisant préféré selon l'invention est $H_3PO_4$ ou (surtout lorsque la source L fournit des ions de $Mo^{VI}$) un acide pyridinecarboxylique.
Les acides pyridinecarboxyliques sont des substances de formule:

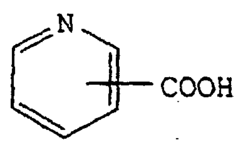

à savoir l'acide 2-pyridinecarboxylique (acide picolinique), l'acide 3-pyridinecarboxylique (acide nicotinique) et l'acide 4-pyridinecarboxylique (acide isonicotinique).
Quand la source L d'ions fournit des ions $Ag^+$ ou $Ag^+/Ag^{2+}$, il est intéressant d'ajouter, les cas échéant, une faible quantité de gélatine à l'agent stabilisant acide du composant D.
De façon avantageuse, le rapport pondéral de l'agent stabilisant au peroxyde d'hydrogène sera compris entre 0,0005/1 et 0,025/1. De façon pratique, ce rapport pondéral sera encore plus avantageusement compris entre 0,0008/1 et 0,005/1 et mieux de l'ordre 0,001/1.

- Les autres additifs

A la composition aqueuse (I) selon l'invention, on peut en outre incorporer l'un au moins des composants suivants :

(E) agent mouillant ;
(F) agent anticorrosion ; et,
(G) parfum.

L'agent mouillant qui intervient ici est (i) un composé tensioactif amphiphile, amphotère, anionique ou non ionique, tel que les polyols convenant en particulier pour contact alimentaire et, le cas échéant, convenant pour administration orale avec l'eau de boisson à la dose considérée d'utilisation, ou (ii) un mélange de tels composés. Convient à cet effet en tant qu'agent mouillant amphotère le produit commercialisé sous la nomenclature de TEGOL® 2000.
Dans la composition aqueuse (I) selon l'invention, le rapport pondéral de l'agent mouillant au peroxyde d'hydrogène sera avantageusement compris entre 0,00005/1 et 0,01/1. De façon pratique, ce rapport pondéral sera encore plus avantageusement de l'ordre de 0,005/1.
Il est recommandé d'incorporer dans la composition aqueuse (I) selon l'invention un agent anticorrosion qui, à la dose utilisée, convient pour contact alimentaire et/ou administration orale avec l'eau de boisson. En tant qu'agents anticorrosion utilisables à cet effet, on peut citer notamment les acides aminophosphoniques tels que décrits dans FR-A-2321302 précité, leurs sels de sodium, de potassium, d'ammonium et d'alkanolamine et leurs mélanges. Sont

particulièrement adaptés à la composition aqueuse (I) selon l'invention les acides hydroxyéthanediphosphonique, diméthylaminométhanediphosphonique, éthylènediamino-tétrakis(méthylènephosphonique), leurs sels de Na, K, $NH_4^+$ ou d'alcanolamine et leurs mélanges. Convient également en tant qu'agent anticorrosion le 1,2,3-benzotriazole.

En pratique l'agent anticorrosion sera présent dans la composition aqueuse (I) à une concentration faible. S'il est utilisé, ledit agent anticorrosion interviendra notamment selon une quantité telle que le rapport pondéral dudit agent anticorrosion au peroxyde d'hydrogène soit compris entre 0,00005/1 et 0,03/1, et de préférence entre 0,001/1 et 0,005/1.

Comme indiqué dans WO-A-96/18301, il est judicieux de limiter la corrosion de surfaces métalliques (principalement en fer ou en cuivre) en contact très prolongé avec la composition aqueuse (I) de l'invention à une valeur inférieure à 200 μm/an.

Comme la corrosion des surfaces métalliques se traduit principalement par un phénomène dit de "piqûres", il est opportun d'éviter la formation desdites piqûres où se logeraient et se développeraient les germes que l'on veut éradiquer.

Le composant parfum du point (G) interviendra dans la composition aqueuse décontaminante selon une quantité inférieure ou égale à celle de l'agent anticorrosion du point (F).

L'eau qui entre dans la composition décontaminante selon l'invention est avantageusement une eau purifiée, à savoir de l'eau distillée, de l'eau déminéralisée ou mieux de l'eau désionisée. De façon préférée, l'eau désionisée sera ici une eau ayant une résistivité supérieure à $10^5$ Ω/cm et mieux supérieure ou égale à $10^6$ Ω/cm.

L'eau utilisée pour les éventuelles dilutions de ladite composition décontaminante selon l'invention sera avantageusement de l'eau purifiée comme indiqué ci-dessus.

Le pH de la composition aqueuse selon l'invention est (avant dilution puis utilisation) en général compris entre 1,2 et 5 et mieux entre 1,5 et 4. Il est régulé au moyen du composant D préféré : $H_3PO_4$ ou acide pyridinecarboxylique.

- <u>La composition aqueuse (I)</u>

De façon préférée, la composition aqueuse (I) selon l'invention, eu égard à ce qui précède, renferme dans de l'eau :

(A) au plus 8 % en poids de $H_2O_2$ ;
(B) un mélange $RCO_2H/RCO_3H$ (où R est $CH_3$ ou $CH_3CH_2$), selon un rapport pondéral dudit mélange $RCO_2H/RCO_3H$ au peroxyde d'hydrogène de 0,15/1 à 0,85/1 et de préférence de 0,5/1 à 0,7/1 ;
(C) une source L fournissant des ions métalliques choisis parmi l'ensemble constitué par :

(1) les ions d'un métal M qui sont des ions de $Ag^{II}$, $Ag^{III}$, $V^{V}$, $Nb^{V}$, $Ta^{V}$, $Mo^{VI}$, $W^{VI}$, $Co^{III}$, $In^{III}$ ou $Tl^{III}$,
(2) les associations d'ions $Ag^+$ et d'ions dudit métal M, et
(3) leurs mélanges,
la source L, qui fournit des ions de $Ag^{II}$ ou des ions de $Ag^{II}$ + $Ag^{III}$, étant telle que, à partir de 100 g d'argent (i.e. 157 g de $AgNO_3$) et 1 litre d'eau déminéralisée, la concentration en ions $Ag^+ + Ag^{2+}$ ou $Ag^+ + Ag^{2+} + Ag^{3+}$ dans la solution résultante donne un potentiel d'oxydo-réduction supérieur à 1 000 mV,

selon un rapport pondéral de ladite source L au peroxyde d'hydrogène de 0,0005/1 à 0,015/1 et de préférence de 0,0008/1 à 0,005/1 ; et,
(D) un agent stabilisant, selon un rapport pondéral dudit agent stabilisant au peroxyde d'hydrogène de 0,0005/1 à 0,015/1 et de préférence de 0,0008/1 à 0,005/1.

De façon encore plus pratique, on préconise une composition standard (i.e. composition mère) qui est destinée à être diluée au moment de l'utilisation. Cette composition standard renferme :

(A) 7,5-7,9 % en poids de $H_2O_2$ ;
(B) 4,5 à 4,8 % en poids d'un mélange $CH_3CO_3H + CH_3CO_2H$ ;
(C) 0,005 à 0,01 % en poids de la source L ;
(D) 0,0005 à 0,01 % en poids de $H_3PO_4$ ou d'acide pyridinecarboxylique ;
(E) le cas échéant 0,0075 à 0,04 % en poids d'agent tensioactif ;
(F) le cas échéant 0,003 à 0,04 % en poids d'agent anticorrosion ;
(G) le cas échéant un parfum ; et,

de l'eau (distillée, déminéralisée ou désionisée) en complément jusqu'à 100 % en poids.

Il peut être avantageux, notamment dans le cadre du traitement de nettoyage, de décapage et/ou de passivation d'une surface métallique ou dans le cadre du traitement de nettoyage et/ou de décapage d'une surface non métallique, que le rapport pondéral C/D soit inférieur à 1/1.

Par ailleurs, quand le composant C est un mélange d'ions $Ag^+$ et d'ions de M, il suffit, selon l'invention, que les ions métalliques de M soient à l'état de traces pour que les effets bénéfiques se manifestent.

- Préparation de la composition (I)

Le procédé de préparation de la composition aqueuse (I) selon l'invention, qui est donné ci-dessus et comprend les étapes (a)-(f), est mis en oeuvre directement quand la source L ne contient pas d'ions $Ag^{2+}$ (ou $Ag^{2+}/Ag^{3+}$). Dans ce cas, ladite composition peut être élaborée en suivant l'enseignement de GB-A-2189394 (pour obtenir une concentration élevée en $H_2O_2$), d'une part, puis en diluant (pour une concentration en $H_2O_2$ inférieure ou égale à 8 % en poids et mieux inférieure ou égale à 7,9 % en poids) avec de l'eau distillée, déminéralisée ou désionisée en vue du transport, d'autre part. En variante, ladite composition peut être élaborée directement sous forme de composition standard.

Quand la source L fournit des ions $Ag^{2+}$(ou $Ag^{2+}/Ag^{3+}$), l'étape (a) est alors remplacée par les étapes (1°) et (2°) pour faire appel à une source d'ions $Ag^+$, que l'on oxyde en ions $Ag^{2+}$. Cette oxydation est mise en oeuvre (i) au moyen d'un persulfate, de préférence le persulfate de sodium ou le persulfate d'ammonium, en tant qu'agent oxydant, et (ii) à l'abri de la lumière blanche pour éviter la réduction par les UV des ions argent en argent métallique qui précipite *in situ.* L'oxydation $Ag^+ \rightarrow Ag^{2+} + e$ sera avantageusement réalisée dans l'obscurité (i.e. dans des réacteurs opaques) ou en lumière rouge.

La filtration prévue *in fine* de l'étape (2°) a pour objet d'éliminer l'insoluble susceptible d'être présent dans le milieu réactionnel. La présence d'un insoluble se manifeste principalement quand le persulfate utilisé est le persulfate d'ammonium. De façon avantageuse, cette filtration est effectuée au moyen d'une membrane filtrante ayant des pores de 3 $\mu$m environ de diamètre.

A l'étape (3°) - i.e. l'étape (b) du procédé général - les ions argent obtenus (en général un mélange de $Ag^+$, de $Ag^{2+}$ et, le cas échéant, de $Ag^{3+}$) sont stabilisés de préférence au moyen de $H_3PO_4$ (en solution aqueuse à 70-85 % en poids) ou d'un acide pyridinecarboxylique.

De façon encore plus avantageuse, une faible quantité de gélatine sera ajoutée avant, pendant ou après l'addition de $H_3PO_4$ ou d'un acide pyridinecarboxylique.

Comme indiqué plus haut, si elle contient des ions $Ag^{2+}$ ou $Ag^{2+} + Ag^{3+}$, la composition aqueuse selon l'invention pourra être élaborée soit sous forme de solution standard soit sous forme de solution ayant une teneur élevée en $H_2O_2$ avant dilution pour le transport.

Un des modes préférés (mode A) de mise en oeuvre du procédé de préparation de la composition (I) selon l'invention, quand la source L fournit des ions de $V^V$, $Nb^V$, $Ta^V$, $Mo^{VI}$, $W^{VI}$, $Co^{III}$, $In^{III}$ ou $Tl^{III}$, comprend les étapes suivantes (pour l'obtention d'une composition standard contenant 7,5-7,9 % en poids de $H_2O_2$) consistant à :

(a) préparer une solution d'un sel de $V^V$, $Nb^V$, $Ta^V$, $Mo^{VI}$, $W^{VI}$, $Co^{III}$, $In^{III}$ ou $Tl^{III}$ (avantageusement en milieu acide) dans une portion de la quantité d'eau totale requise pour réaliser ladite composition aqueuse désinfectante ;
(b) introduire dans la solution résultante ainsi obtenue une solution aqueuse d'acide phosphorique contenant 70 à 85 % en poids de $H_3PO_4$ ou une solution aqueuse d'acide pyridinecarboxylique ;
(c) introduire la solution résultante, ainsi obtenue, dans une solution aqueuse de peroxyde d'hydrogène contenant 50 à 60 % en poids de $H_2O_2$, sous agitation, à une température comprise entre 0°C et 25°C (de préférence à une température comprise entre 4°C et 15°C), et avec un débit d'introduction de la solution obtenue à l'étape (b) compris entre 3 et 6 l/h ;
(d) introduire dans la solution résultante, ainsi obtenue, la substance acide $CH_3CO_2H$, sous agitation, à une température comprise entre 0°C et 25°C (de préférence à une température comprise entre 4°C et 15°C), et avec un débit d'introduction de la substance acide $CH_3CO_2H$ compris entre 3 et 6 l/h ;
(e) laisser reposer la solution résultante, ainsi obtenue, pendant 48 h (de préférence à l'obscurité), à une température comprise entre 0°C et 25°C (de préférence à une température comprise entre 4°C et 15°C), pour que l'équilibre

$$H_2O_2 + CH_3CO_2H \Leftrightarrow CH_3CO_3H + H_2O$$

s'établisse, et,
(f) ajouter l'eau restante pour compléter jusqu'à 100 % en poids.

A l'étape (d) de la préparation de cette composition, l'acide $CH_3CO_2H$ peut être introduit sous forme de solution

aqueuse.

Un autre mode préféré (mode B) de mise en oeuvre du procédé de préparation de la composition (I) selon l'invention, quand la source L fournit des ions $Ag^{2+}$ (avec le cas échéant des ions $Ag^{3+}$), comprend les étapes suivantes (pour l'obtention d'une composition standard contenant 7,5-7,9 % en poids de $H_2O_2$) consistant à :

(1°) dissoudre $AgNO_3$ dans de l'eau déminéralisée ayant une résistivité de $10^6$ Ω/cm (à raison de 157 g de nitrate d'argent - i.e. 100 g d'argent - pour un litre d'eau déminéralisée), le potentiel rédox de la solution ainsi obtenue étant d'environ 600 mV ;
(2°) oxyder au moins une portion des ions $Ag^+$ de la solution précédente en $Ag^{2+}$ ou en $Ag^{2+}$ + $Ag^{3+}$ par addition de persulfate de sodium ou de persulfate d'ammonium, de façon à obtenir un potentiel rédox supérieur ou égal à 1000 mV (notamment un potentiel rédox supérieur ou égal à 1600 mV) ; filtrer (sur membrane ayant des pores de 3 μm de diamètre) ;
(3°) stabiliser la solution résultante ainsi obtenue au moyen d'une solution aqueuse d'acide phosphorique contenant 70 à 85 % en poids de $H_3PO_4$ [à raison de 10 à 100 ml de solution aqueuse de $H_3PO_4$ à 70 à 85 % en poids, pour 1 litre d'eau déminéralisée utilisé à l'étape (1°)] ou d'une solution aqueuse d'acide pyridinecarboxylique [après agitation pendant 24 h à température ambiante, la solution d'ions argent ainsi stabilisée ayant un potentiel rédox supérieur ou égal à 1000 mV] ;
(4°) introduire la solution résultante, ainsi obtenue, dans une solution aqueuse de peroxyde d'hydrogène contenant 50 à 60 % en poids de $H_2O_2$, sous agitation, à une température comprise entre 0°C et 25°C (de préférence à une température comprise entre 4°C et 15°C), et avec un débit d'introduction de la solution obtenue à l'étape (3°) compris entre 3 et 6 l/h ;
(5°) introduire dans la solution résultante, ainsi obtenue, la substance acide $CH_3CO_2H$, sous agitation, à une température comprise entre 0°C et 25°C (de préférence à une température comprise entre 4°C et 15°C), et avec un débit d'introduction de la substance acide $CH_3CO_2H$ compris entre 3 et 6 l/h ;
(6°) laisser reposer la solution résultante, ainsi obtenue, pendant 48 h, à l'obscurité (ou en lumière rouge) à une température comprise entre 0°C et 25°C (de préférence à une température comprise entre 4°C et 15°C), pour que l'équilibre

$$H_2O_2 + CH_3CO_2H \Leftrightarrow CH_3CO_3H + H_2O$$

s'établisse, et,
(7°) ajouter de l'eau pour compléter jusqu'à 100 % en poids.

A l'étape (5°) de la préparation de cette composition standard, l'acide $CH_3CO_2H$ peut être introduit sous forme de solution aqueuse.

- <u>La composition gélifiée (II)</u>

La composition gélifiée (II) conforme à l'invention est obtenue à partir de la composition aqueuse (I) et d'un agent gélifiant particulier, qui est une substance minérale ou une substance organique. Quand cet agent gélifiant est une substance minérale il s'agit avantageusement de silice colloïdale pyrogénée se présentant à l'état sec sous forme de particules ayant une surface massique de 80 à 400 $m^2/g$ et une granulométrie moyenne de 7 à 20 nm. Parmi les silices, qui conviennent à cet effet, on peut notamment citer celles qui sont commercialisées par la société dite CABOT sous la nomenclature CAB-O-SIL®, et en particulier les produits CAB-O-SIL® LM-150, CAB-O-SIL® M-5, CAB-O-SIL® EH-5 et CAB-O-SIL® M-7D qui sont hydrophiles.

Pour l'obtention d'une composition gélifiée (100 parties en poids) contenant de la silice pyrogénée on procède comme suit :

- mettre en contact la composition (I) (50 à 99 parties en poids) et la silice colloïdale pyrogénée (50 à 1 parties en poids),
- agiter pendant 3-10 minutes avec une vitesse angulaire de l'agitateur de 1500 à 2000 tours/minute), puis
- laisser reposer à température ambiante.

Quand l'agent gélifiant est une substance organique, il s'agit avantageusement d'un matériau polyacrylique comme indiqué plus haut. Dans ce cas, la composition gélifiée (100 parties en poids) qui contient ledit matériau polyacrylique sera préparée comme suit :

- mettre en contact la composition (I) (90 à 99,7 parties en poids) et le matériau gélifiant organique (10 à 0,3 parties en poids),
- agiter pendant 10-20 minutes avec une vitesse angulaire de l'agitateur de 1 000 à 2 000 tours/minute), puis
- laisser reposer à la température ambiante.

La composition gélifiée (II) contenant un matériau gélifiant organique polyacrylique ne peut être utilisée qu'à un pH supérieur ou égal à 3,5, en pratique elle sera utilisée à un pH de 3,5 à 7 et de préférence à pH 5. Par ailleurs, elle convient pour la réalisation de gels transparents prisés du public en dermatologie et en cosmétologie.

En revanche, la composition gélifiée (II) contenant de la silice pyrogénée sera avantageusement utilisée à un pH de 1,5 à 4.

Le gel obtenu a des propriétés thixotropiques : il devient fluide sous contrainte telle qu'agitation, frottement ou cisaillement et se prend en masse rapidement (en moins de 5 minutes) quand l'agitation, le frottement ou le cisaillement cesse.

Par ailleurs, ce gel est stable en milieu acide ou faiblement alcalin ; il est également stable jusqu'à la température de dégradation de ses constituants.

En fonction de la teneur en eau, ledit gel est réalisable dans une large gamme de viscosités.

En raison de ses propriétés thixotropiques, la composition gélifiée (II) selon l'invention est particulièrement adaptée à la dépollution des huiles et graisses vieillies (qui sont des milieux nutritifs de plusieurs microorganismes susceptibles d'être pathogènes) *in situ* dans les dispositifs et installations industriels.

- Les utilisations

Les utilisations de la composition aqueuse oxydante (I) selon l'invention comprennent (i) celles envisagées dans publication WO-A-96/18301 précitée (voir de la page 20 ligne 11 à la page 22 ligne 15) en ce qui concerne notamment la désinfection de l'eau pour la rendre potable, l'hygiène des locaux industriels et des piscines, la stérilisation à froid des instruments chirurgicaux et dentaires, la protection des plantes et des récoltes vis-à-vis des bactéries, moisissures, virus et parasites, la protection des poissons, des crustacés et des coquillages vis-à-vis des algues pathogènes telles que les ***Euglena,*** et la dépollution des sites miniers, et (ii) les utilisations nouvelles qui ont trait au nettoyage, au décapage et/ou à la passivation des surfaces métalliques (notamment les surfaces en acier ou en aluminium) ou non métalliques (notamment les surfaces en plastique ou céramique, les surfaces en plastique comprenant ici celles des panneaux de revêtement de sols ou de murs qui sont exposées et généralement conçues en PVC, polyacrylate, polycarbonate ou autre).

De façon avantageuse la composition (I) destinée à la désinfection et l'hygiène sera obtenue par dilution d'une composition standard de façon à avoir une concentration en $H_2O_2$ de 1 à 2 % en poids.

De façon également avantageuse la composition (I) destinée à la dépollution des sites miniers aura une teneur en $H_2O_2$ de 4 à 7,9 % en poids et sera diluée au moment de l'emploi jusqu'à une concentration finale d'utilisation inférieure ou égale à 1/100 (i.e. une teneur finale en $H_2O_2$ inférieure ou égale à 0,04 % en poids).

Dans le domaine du traitement de surface, la composition (I) aura avantageusement une teneur en $H_2O_2$ comprise entre 1 et 7,9 % en poids et comportera un rapport pondéral C/D inférieur à 1/1 et supérieur à 1/2.

La composition gélifiée (II) selon l'invention offre l'avantage de fournir, au voisinage de l'article ou de la surface à traiter, une source de $H_2O_2$ et d'ions provenant de L qui est stabilisée et qui assure une longue durée d'action.

La composition gélifiée (II) est particulièrement utile vis-à-vis des surfaces qui ne sont pas horizontales, c'est-à-dire des surfaces verticales, courbes ou inclinées. Elle est tout particulièrement adaptée à la protection des constructions vis-à-vis de germes susceptibles de se développer dans les cavités des parois extérieures des bâtiments exposées à la poussière et aux nuisances des oiseaux, d'une part, et au décapage et à la passivation des cuves ou réacteurs métalliques, d'autre part.

On préconise donc selon l'invention l'utilisation de la composition gélifiée II, d'une part, en tant que produit germicide pour la préparation d'un médicament destiné à un usage en thérapeutique vis-à-vis des affections buccales, notamment celle du parondonte, dues à des bactéries et/ou des champignons, et d'autre part, en tant que produit cosmétique notamment sous forme de dentifrice, pour l'hygiène buccale.

Dans le domaine dentaire, on a observé que la composition gélifiée (II) intervient en tant qu'agent hémostatique, supprime l'administration d'antibactériens et/ou d'antifongiques, inhibe la catalase, et assure la passivation des surfaces métalliques des bridges (ladite passivation empêchant le dépôt de bactéries et de champignons pendant une longue durée de temps) de même que la protection temporaire des surfaces plastiques ou céramiques vis-à-vis desdites bactéries et champignons (leur dépôt étant ainsi empêché). Cette composition gélifiée est appropriée à la préparation de pâtes de dentifrice renfermant 1 à 2 % en poids de peroxyde d'hydrogène.

D'autres avantages et caractéristiques de l'invention seront mieux compris à la lecture qui va suivre d'exemples de réalisation et d'essais comparatifs. Bien entendu, l'ensemble de ces éléments n'est nullement limitatif mais est

fourni à titre d'illustration. Par commodité, quand il s'agit du composant (C) on a indiqué ci-après dans Ex 1-Ex 6 la source d'ions L, sa proportion pondérale et les ions qu'elle fournit.

***Exemple 1***

On prépare selon le mode B donné ci-dessus, une composition standard contenant :

| | |
|---|---|
| $H_2O_2$ | 7,8 % en poids |
| mélange $CH_3CO_2H$ + $CH_3CO_3H$ | 4,8 % en poids |
| $AgNO_3$ (source de $Ag^+/Ag^{2+}$) | 0,008 % en poids |
| $H_3PO_4$ ou acide pyridinecarboxylique | 0,008 % en poids |
| agent tensioactif (mouillant anionique) | 0,04 % en poids |
| $H_2O$ (déminéralisée) en complément jusqu'à | 100 % en poids |

***Exemple 2***

On prépare selon le mode A donné ci-dessus, une composition standard contenant :

| | |
|---|---|
| $H_2O_2$ | 7,8 % en poids |
| mélange $CH_3CO_2H$ + $CH_3CO_3H$ | 4,8 % en poids |
| $Na_2MoO_4$ (source de $MoO_4^{2-}$) | 0,008 % en poids |
| acide pyridinecarboxylique | 0,008 % en poids |
| agent tensioactif (mouillant anionique) | 0,04 % en poids |
| $H_2O$ (déminéralisée) en complément jusqu'à | 100 % en poids |

Quand la source L d'ions fournit des ions de $Mo^{VI}$, il est important de ne pas utiliser $H_3PO_4$ en tant qu'agent stabilisant. Il faut éviter la mise en contact de $H_3PO_4$ avec $MoO_4^{2-}$ qui donnerait un complexe du type phosphonomolybdate qui est insoluble.

***Exemple 3***

On prépare selon le mode A donné ci-dessus, une composition standard contenant :

| | |
|---|---|
| $H_2O_2$ | 7,8 % en poids |
| mélange $CH_3CO_2H$ + $CH_3CO_3H$ | 4,8 % en poids |
| $NH_4VO_3$ (source de $VO_3^-$, $VO_2^+$ ou $V^{5+}$) | 0,008 % en poids |
| $H_3PO_4$ ou acide pyridinecarboxylique | 0,008 % en poids |
| agent tensioactif (mouillant anionique) | 0,04 % en poids |
| $H_2O$ (déminéralisée) en complément jusqu'à | 100 % en poids |

***Exemple 4***

On prépare selon le mode A donné ci-dessus, une composition standard contenant :

| | |
|---|---|
| $H_2O_2$ | 7,8 % en poids |
| mélange $CH_3CO_2H$ + $CH_3CO_3H$ | 4,8 % en poids |
| $Na_2WO_4$ (source de $WO_4^{2-}$) | 0,008 % en poids |
| $H_3PO_4$ ou acide pyridinecarboxylique | 0,008 % en poids |
| agent tensioactif (mouillant anionique) | 0,04 % en poids |
| $H_2O$ (déminéralisée) en complément jusqu'à | 100 % en poids |

***Exemple 5***

On prépare la composition standard de l'exemple 5 à partir d'un mélange pondéral 1/1 des compositions des

exemples 1 et 2. Cette composition standard contient :

| | |
|---|---|
| $H_2O_2$ | 7,8 % en poids |
| mélange $CH_3CO_2H$ + $CH_3CO_3H$ | 4,8 % en poids |
| $AgNO_3$ (source de $Ag^+$/$Ag^{2+}$) | 0,004 % en poids |
| $Na_2MoO_4$ (source de $MoO_4^{2-}$) | 0,004 % en poids |
| acide pyridinecarboxylique | 0,008 % en poids |
| agent tensioactif (mouillant anionique) | 0,04 % en poids |
| $H_2O$ (déminéralisée) en complément jusqu'à | 100 % en poids |

***Exemple comparatif CP1***

Comme produit de comparaison, on a fait appel à la composition de l'exemple 1 du document antérieur WO-A-96/18301. Sa formulation est la suivante :

| | |
|---|---|
| $H_2O_2$ | 8 % en poids |
| mélange $CH_3CO_2H$ + $CH_3CO_3H$ | 4,8 % en poids |
| $AgNO_3$ (source de $Ag^+$) | 0,008 % en poids |
| $H_3PO_4$ | 0,008 % en poids |
| agent tensioactif (mouillant anionique) | 0,04 % en poids |
| $H_2O$ (déminéralisée) en complément jusqu'à | 100 % en poids |

***Exemple 6***

A partir d'un mélange pondéral 1/1 des compositions de Ex 2 et CP1, on obtient une composition standard (après avoir remplacé dans CP1 l'acide phosphorique par un acide pyridinecarboxylique) ayant pour formulation :

| | |
|---|---|
| $H_2O_2$ | 7,9 % en poids |
| mélange $CH_3CO_2H$ + $CH_3CO_3H$ | 4,8 % en poids |
| $AgNO_3$ (source de $Ag^+$) | 0,004 % en poids |
| $Na_2MoO_4$ (source de $MoO_4^{2-}$) | 0,004 % en poids |
| acide pyridinecarboxylique | 0,008 % en poids |
| agent tensioactif (mouillant anionique) | 0,04 % en poids |
| $H_2O$ (déminéralisée) en complément jusqu'à | 100 % en poids |

***Essais comparatifs I***

L'activité germicide de Ex 1-Ex 6 et CP1 a été déterminée vis-à-vis de trois souches bactériennes (1-3) et d'une souche de moisissure (4) qui sont pathogènes chez l'homme (1-2) ou phytopathogènes (3-4) notamment pour la tomate de plein champ, à savoir :

(1) ***Staphylococcus aureus,***
(2) ***Speudomonas aerugina,***
(3) ***Speudomonas syringae,*** et
(4) ***Alternaria solani.***

Si $[S]_0$ est la concentration d'une souche S à l'instant 0 et $[S]_T$ celle de la même souche à l'instant T (T = 1h pour les bactéries et T = 2h pour les moisissures), l'activité germicide est mesurée par la différence :

$$\Delta = \log[S]_0 - \log[S]_T$$

un produit étant actif quand il réduit le nombre de souches vivantes de telle façon que $\Delta \geq 4$ pour les bactéries et $\Delta \geq 3$ pour les moisissures.

Les résultats obtenus pour $\Delta$ consignés dans le tableau I ci-après mettent en évidence que CP1 à la dilution

de 1/5 est efficace vis-à-vis des bactéries mais est pratiquement dépourvu d'activité sur la souche de ***Alternaria solani.*** Ces résultats mettent également en évidence que, à la même dilution de 1/5, Ex 1-Ex 6 sont (i) efficaces sur les bactéries et les moisissures, et (ii) plus actifs que CP1.

***Exemple 7***

A un litre de la composition aqueuse de Ex 1, on ajoute sous forte agitation (2000 tours/minute) 10 g de silice pyrogénée CAB-O-SIL® M-5 pendant 6 minutes. On laisse reposer et on obtient un gel ayant une viscosité de $10^6$ Pa.s.

***Essais comparatifs II***

On a comparé l'action de la composition aqueuse CP1 avec le gel de Ex 7. Les deux produits ont été appliqués pendant 4 heures sur une toiture en alliage d'aluminium anodisé exposée à l'ouest et recouverte de mousses et lichens. Le traitement avec CP1 s'est révélé inefficace à cause du trop faible temps de contact dû à l'évaporation de la solution. En revanche, l'ensemble des mousses et lichens a été éliminé par un simple rinçage à l'eau courante à l'issue des 4 heures d'expérimentation.

***Exemple 8***

A un litre de la composition aqueuse de Ex 1, on ajoute sous forte agitation (2000 tours/minute) 100 g de silice pyrogénée CAB-O-SIL® M-5 et 500 ml d'une solution aqueuse d'acide phosphorique contenant 75 % en poids de $H_3PO_4$, pendant 6 minutes. On laisse reposer et on obtient un gel ayant une viscosité de $10^7$ Pa.s.
Le gel ainsi obtenu est fortement thixotropique.

***Essais III***

Le gel de Ex 8, appliqué sur une hotte de cuisine industrielle en acier inoxydable 316L dépassivée à la suite d'un incendie ayant dégagé des vapeurs riches en chlore, et laissé en contact pendant 3 heures a permis à l'acier inoxydable 316 L d'être complètement décontaminé et de retrouver son potentiel de passivité.

***Exemple 9***

On dilue avec de l'eau déminéralisée la composition aqueuse de Ex 1 de façon à obtenir une teneur en $H_2O_2$ de 1 à 2 % en poids. La solution résultante est ensuite gélifiée selon le procédé de l'exemple 7 ci-dessus.

***Essais IV***

Le gel de Ex 9 a été appliqué une fois par jour pendant 5 jours sur les dents et la muqueuse du parondonte située au voisinage des dents, chez des patients atteints de plaque dentaire et/ou de mycose buccale et ayant une mauvaise haleine. On a constaté par des biopsies de la muqueuse du parondonte une diminution totale *in situ* des bactéries et des champignons responsables de la plaque dentaire et respectivement des mycoses. Les patients traités n'avaient plus une mauvaise haleine.

***Exemple 10***

On dilue avec de l'eau déminéralisée la composition aqueuse de Ex 1 de façon à obtenir une teneur en $H_2O_2$ de 4 % en poids. La solution résultante est ensuite gélifiée selon le procédé de l'exemple 8 ci-dessus.

***Essais V***

Le gel de Ex 10 a été appliqué sur des cuves en acier inoxydable utilisées pour la fabrication du champagne. Statistiquement on sait que le contenu d'une cuve sur cinq ou six est perdu du fait (i) du développement de germes inappropriés et /ou (ii) de la dégradation de la paroi interne. Par traitement de la paroi interne des cuves pendant 5h avec le gel de Ex 10 puis rinçage à l'eau, une fois avant les vendanges et une fois après la vidange des cuves qui suit la fermentation, on maintient en activité l'ensemble des cuves en évitant la perte classique sus-visée.

***Essai VI***

On dilue extemporanément (i.e. au moment de l'emploi) chacune des compositions de Ex 1, Ex 2, Ex 5 et CP1 de façon à obtenir une dilution de 1/5. On verse 50 ml de chacune des solutions ainsi diluées dans des lots de récipients transparents en verre et immerge dans ces solutions, à l'instant T = 0, une prothèse dentaire qui (a) comporte des surfaces métalliques, plastiques et/ou céramiques, (b) est infestée par des bactéries et/ou moisissures et (c) est souillée par des traces de tissus (sang coagulé, muqueuse, chair) et d'adhésif. On laisse reposer les récipients ouverts. On observe les prothèses des lots de récipients aux instants T = 1h, T = 2h, T = 15j et T = 90j.

On constate que, à T = 1h, les prothèses immergées dans les compositions diluées de Ex 1, Ex 2 et Ex 5 sont propres et essentiellement dépourvues de bactéries et de moisissures ; à t = 2h, les prothèses immergées dans les compositions diluées de Ex 1, Ex 2 et Ex 5 sont propres et essentiellement dépourvues de bactéries et de moisissures, et les prothèses immergées dans la composition diluée de CP1 sont propres, dépourvues de bactéries mais sont toujours contaminées par des moisissures (population $\geq 10^4$ germes/ml) ; à T = 15j, toutes les prothèses sont propres et dépourvues de bactéries, celles immergées dans la composition diluée de CP1 ayant une population en moisissures supérieure à $10^4$ germes/ml alors que celles traitées dans les dilutions de Ex 1, Ex 2 et Ex 5 sont dépourvues de moisissures ; à T = 90j, seules les prothèses traitées dans les dilutions de Ex 1, Ex 2 et Ex 5 sont propres et dépourvues de bactéries et moisissures, celles traitées dans la dilution de CP1 sont à nouveau contaminées par des bactéries et des moisissures.

En bref, après une heure de traitement avec Ex 1, Ex 2 et Ex 5, chaque prothèse est considérée comme "étant toute neuve et réutilisable". On constate par ailleurs que Ex 1, Ex 2 et Ex 5, à la différence de CP1, empêchent la fixation en surface des bactéries et moisissures.

Tableau I

| *Activité germicide* $\Delta = \log[S]_0 - \log[S]_T$ | | | | |
|---|---|---|---|---|
| Produit | Souches | | | |
| | (a) | (b) | (c) | (d) |
| CP1 | 4,56 | 5,38 | 3,97 | 1,61 |
| Ex 1 | 6,27 | 6,14 | 5,23 | 4,42 |
| Ex 2 | 6,25 | 6,17 | 5,25 | 4,44 |
| Ex 3 | 5,0 | 5,19 | 4,67 | 4,45 |
| Ex 4 | 6,27 | 5,17 | 5,30 | 4,43 |
| Ex 5 | 6,25 | 5,67 | 5,27 | 4,42 |
| Ex 6 | 5,37 | 5,70 | 5,27 | 4,42 |
| Notes : (a) ***Staphylococcus aureus*** (b) ***Pseudomonas aerugina*** (c) ***Pseudomonas syringae*** (d) ***Alternaria solani*** | | | | |

***Exemple 11***

On prépare une composition dermatologique ou cosmétique à partir de la formulation suivante :

| | |
|---|---|
| $H_2O_2$ | 1 partie en poids |
| mélange $CH_3CO_2H$ + $CH_3CO_3H$ | 0,5 partie en poids |
| $CH_3$-CH=CH-CH=CH-$CO_2H$ | 0,1 partie en poids |
| acide pyridinecarboxylique | 0,004 partie en poids |
| $Na_2MoO_4$ (source de $MoO_4^{2-}$) | 0,004 partie en poids |
| agent tensioactif (mouillant non ionique) | 0,02 partie en poids |
| $H_2O$ (déminéralisée) en complément jusqu'à | 95,5 parties en poids |

Les ingrédients sont introduits dans une portion de l'eau déminéralisée requise dans l'ordre suivant : (i) $H_2O_2$,

(ii) $CH_3CO_2H/CH_3CO_3H$ + $CH_3$-CH=CH-CH=CH-$CO_2H$ + acide pyridine-carboxylique, (iii) $Na_2MoO_4$ puis (iv) eau déminéralisée jusqu'à un poids total de 95,5 parties en poids.

***Exemple 12***

On prépare une composition gélifiée contenant un matériau organique polyacrylique en tant qu'agent gélifiant, à partir de la formulation suivante :

| | |
|---|---|
| $H_2O_2$ | 1 % en poids |
| mélange $CH_3CO_2H$ + $CH_3CO_3H$ | 0,5 % en poids |
| $CH_3$-CH=CH-CH=CH-$CO_2H$ | 0,1 % en poids |
| acide pyridinecarboxylique | 0,004 % en poids |
| $Na_2MoO_4$ (source de $MoO_4^{2-}$) | 0,004 % en poids |
| NaOH (jusqu'à pH 5) | 1 % en poids |
| agent tensioactif (mouillant non ionique) | |
| CARBOPOL® ETD 2623 | 0,5 % en poids |
| $H_2O$ (déminéralisée) en complément jusqu'à | 100 % en poids |

Cette composition est préparée par agitation (à 800-1000 tours/minute) du CARBOPOL® 2623 dans une portion de l'eau déminéralisée requise, maintien de cette agitation pendant 15 minutes, chauffage à 45°C jusqu'à dissolution complète, ajout dans l'ordre de (i) $H_2O_2$, (ii) $CH_3CO_2H/CH_3CO_3H$, $CH_3$-CH=CH-CH=CH-$CO_2H$ puis acide pyridinecarboxylique, (iii) $Na_2MoO_4$ puis agent tensioactif, agitation pendant 15 minutes (à 1000 tours/minute), ajustement du pH à 5 par NaOH, et (iv) eau déminéralisée jusqu'à 100 % en poids. La viscosité Brookfield sous 20 tours/minute de cette composition gélifiée est de 15 Pa.s (i.e. 15 000 cP).

## Revendications

1. Composition aqueuse oxydante (I), qui est utile notamment dans le domaine de la désinfection, de l'hygiène et de la dépollution, d'une part, et dans le domaine du traitement de surface (en particulier pour le nettoyage, le décapage et/ou la passivation), d'autre part, ladite composition, qui contient dans de l'eau du peroxyde d'hydrogène, un mélange $RCO_2H/RCO_3H$ (où R est un reste aliphatique en $C_1$-$C_6$ à chaîne hydrocarbonée linéaire ou ramifiée, saturée ou insaturée), et un agent stabilisant qui est un acide, et, le cas échéant, des ions $Ag^+$, étant **caractérisée en ce qu'**elle renferme des ions d'au moins un métal M qui sont des ions de $Ag^{II}$, $Ag^{III}$, $V^V$, $Nb^V$, $Ta^V$, $Mo^{VI}$, $W^{VI}$, $Co^{III}$, $In^{III}$ ou $Tl^{III}$, la source L, qui fournit des ions de $Ag^{II}$ ou des ions de $Ag^{II}$ + $Ag^{III}$, étant telle que, à partir de 100 g d'argent (i.e. 157 g de $AgNO_3$) et 1 litre d'eau déminéralisée, la concentration en ions $Ag^+$ + $Ag^{2+}$ ou $Ag^+$ + $Ag^{2+}$ + $Ag^{3+}$ dans la solution résultante donne un potentiel d'oxydo-réduction supérieur à 1 000 mV.

2. Composition suivant la revendication 1, **caractérisée en ce qu'**elle renferme dans de l'eau :

   (A) $H_2O_2$ ;
   (B) un mélange $RCO_2H/RCO_3H$ (où R est un reste aliphatique en $C_1$-$C_6$ à chaîne hydrocarbonée linéaire ou ramifiée, saturée ou insaturée) ;
   (C) des ions métalliques provenant d'une source L et choisis parmi l'ensemble constitué par

      (1) les ions d'un métal M qui sont des ions de $Ag^{II}$, $Ag^{III}$, $V^V$, $Nb^V$, $Ta^V$, $Mo^{VI}$, $W^{VI}$, $Co^{III}$, $In^{III}$ ou $Tl^{III}$,
      (2) les associations d'ions $Ag^+$ et d'ions dudit métal M, et
      (3) leurs mélanges ;
      la source L, qui fournit des ions de $Ag^{II}$ ou des ions de $Ag^{II}$ + $Ag^{III}$, étant telle que, à partir de 100 g d'argent (i.e. 157 g de $AgNO_3$) et 1 litre d'eau déminéralisée, la concentration en ions $Ag^+$ + $Ag^{2+}$ ou $Ag^+$ + $Ag^{2+}$ + $Ag^{3+}$ dans la solution résultante donne un potentiel d'oxydo-réduction supérieur à 1 000 mV ; et,

   (D) un agent stabilisant qui est un acide.

3. Composition suivant la revendication 2, **caractérisée en ce que** lesdits ions métalliques sont des ions de $Mo^{VI}$, un mélange d'ions $Ag^+/Ag^{2+}$, un mélange d'ions de $Mo^{VI}/Ag^+$ ou un mélange d'ions de $Mo^{VI}/Ag^+/Ag^{2+}$.

**4.** Procédé pour la préparation d'une composition aqueuse suivant la revendication 1 ou 2, ledit procédé étant **caractérisé en ce qu'**il comprend les étapes consistant à

(a) faire appel à une solution aqueuse d'une source L fournissant les ions métalliques choisis parmi l'ensemble constitué par

(1) les ions d'un métal M qui sont des ions de $Ag^{II}$, $Ag^{III}$, $V^{V}$, $Nb^{V}$, $Ta^{V}$, $Mo^{VI}$, $W^{VI}$, $In^{III}$ ou $Tl^{III}$,
(2) les associations d'ions $Ag^{+}$ et d'ions dudit métal M, et
(3) leurs mélanges ;
la source L, qui fournit des ions de $Ag^{II}$ ou des ions de $Ag^{II}$ + $Ag^{III}$, étant telle que, à partir de 100 g d'argent (i.e. 157 g de $AgNO_3$) et 1 litre d'eau déminéralisée, la concentration en ions $Ag^{+} + Ag^{2+}$ ou $Ag^{+} + Ag^{2+} + Ag^{3+}$ dans la solution résultante donne un potentiel d'oxydo-réduction supérieur à 1 000 mV ;

(b) introduire dans ladite solution l'agent stabilisant ;
(c) introduire ladite solution résultante, ainsi obtenue, dans de l'eau oxygénée ou introduire l'eau oxygénée dans ladite solution résultante ;
(d) introduire dans la solution résultante, ainsi obtenue, une substance acide qui est $RCO_3H$, $RCO_2H$ ou leur mélange $RCO_3H + RCO_2H$ où R est défini comme indiqué ci-dessus ;
(e) laisser reposer la solution résultante, ainsi obtenue, jusqu'à ce que l'équilibre $H_2O_2 + RCO_2H \Leftrightarrow H_2O + RCO_3H$ soit établi ; et,
(f) compléter jusqu'à 100 % en poids avec de l'eau.

**5.** Procédé suivant la revendication 4, pour la préparation d'une composition qui contient des ions $Ag^{2+}$, ledit procédé étant **caractérisé en ce qu'**il comprend les étapes consistant à

(1°) préparer une solution aqueuse d'un composant d'argent, qui intervient en tant que source d'ions $Ag^{+}$ ;
(2°) oxyder au moins une partie des ions $Ag^{+}$ en ions $Ag^{2+}$ ou $Ag^{2+}$+$Ag^{3+}$ au moyen de persulfate, de façon à obtenir un potentiel rédox supérieur ou égal à 1000 mV à partir de 1 litre d'eau déminéralisée et de 157 g de $AgNO_3$, et filtrer pour écarter l'insoluble susceptible d'être présent ;
(3°) introduire dans la solution résultante, ainsi obtenue, l'agent stabilisant ;
(4°) introduire ladite solution résultante, ainsi obtenue, dans de l'eau oxygénée ou introduire l'eau oxygénée dans ladite solution résultante ;
(5°) introduire dans la solution résultante, ainsi obtenue, une substance acide qui est $RCO_3H$, $RCO_2H$ ou leur mélange $RCO_3H + RCO_2H$ où R est défini comme indiqué ci-dessus ;
(6°) laisser reposer la solution résultante, ainsi obtenue, jusqu'à ce que l'équilibre $H_2O_2 + RCO_2H \Leftrightarrow H_2O + RCO_3H$ soit établi ; et,
(7°) compléter jusqu'à 100 % en poids avec de l'eau.

**6.** Composition suivant la revendication 1 ou 2, **caractérisée en ce qu'**elle se présente sous la forme d'une composition gélifiée (II) qui comprend la composition aqueuse selon la revendication 1 ou 2 et un agent gélifiant minéral ou organique.

**7.** Composition suivant la revendication 6, **caractérisée en ce qu'**elle comprend :

($\alpha$) 50 à 99 % en poids de la composition aqueuse (I) selon la revendication 1 ou 2, et
($\beta$) 50 à 1 % en poids de silice colloïdale pyrogénée ayant une surface massique de 80 à 400 $m^2/g$ et une granulométrie moyenne de 7 à 20 nm en tant qu'agent gélifiant.

**8.** Composition suivant la revendication 6, **caractérisée en ce qu'**elle comprend :

($\alpha$) 90 à 99,7 % en poids de la composition aqueuse (I) selon la revendication 1 ou 2, et
($\beta$) 10 à 0,3 % en poids d'un matériau organique polyacrylique en tant qu'agent gélifiant.

**9.** Utilisation de la composition gélifiée suivant l'une quelconque des revendications 6 à 8, **caractérisée en ce que** l'on fait appel à ladite

## Patentansprüche

**1.** Oxidierende wässrige Zusammensetzung (I), die sich insbesondere auf dem Gebiet der Desinfektion, der Hygiene und der Beseitigung von Umweltverschmutzungen einerseits und auf dem Gebiet der Behandlung von Oberflächen (insbesondere zur Reinigung, Abbeizung und/oder Passivierung) andererseits eignet, wobei die Zusammensetzung, die in Wasser Wasserstoffperoxid, ein $RCO_2H/RCO_3H$-Gemisch (worin R einen aliphatischen $C_1$-$C_6$-Rest mit einer gesättigten oder ungesättigten, linearen oder verzweigten Kohlenwasserstoffkette bedeutet) und ein Stabilisierungsmittel, bei dem es sich um eine Säure handelt, und gegebenenfalls $Ag^+$-Ionen enthält, **dadurch gekennzeichnet, dass** sie Ionen mindestens eines Metalls M enthält, bei denen es sich um die Ionen von $Ag^{II}$, $Ag^{III}$, $V^V$, $Nb^V$, $Ta^V$, $Mo^{VI}$, $W^{VI}$, $Co^{III}$, $In^{III}$ oder $Tl^{III}$ handelt, wobei die Quelle L, die die Ionen von $Ag^{II}$ oder die Ionen von $Ag^{II}$ + $Ag^{III}$ liefert, so beschaffen ist, dass ausgehend von 100 g Silber (d. h. 157 g $AgNO_3$) und 1 Liter entmineralisiertem Wasser die Konzentration an den Ionen $Ag^+$ + $Ag^{2+}$ oder $Ag^+$ + $Ag^{2+}$ + $Ag^3$+ in der erhaltenen Lösung ein Redoxpotential von mehr als 1000 mV ergibt.

**2.** Zusammensetzung nach Anspruch 1, **dadurch gekennzeichnet, dass** sie in Wasser folgende Bestandteile enthält:

(A) $H_2O_2$;
(B) ein $RCO_2H/RCO_3H$-Gemisch (worin R einen aliphatischen $C_1$-$C_6$-Rest mit einer gesättigten oder ungesättigten, linearen oder verzweigten Kohlenwasserstoffkette bedeutet);
(C) Metallionen, die von einer Quelle L stammen und aus folgender Gruppe ausgewählt sind:

(1) Ionen eines Metalls M, bei denen es sich um die Ionen $Ag^{II}$, $Ag^{III}$, $V^V$, $Nb^V$, $Ta^V$, $Mo^{VI}$, $W^{VI}$, $Co^{III}$, $In^{III}$ oder $Tl^{III}$ handelt,
(2) Kombinationen von $Ag^+$-Ionen und Ionen des Metalls M und
(3) Gemische davon;

wobei die Quelle L, die die Ionen von $Ag^{II}$ oder die Ionen von $Ag^{II}$ + $Ag^{III}$ liefert, so beschaffen ist, dass ausgehend von 100 g Silber (d. h. 157 g $AgNO_3$) und 1 Liter entmineralisiertem Wasser die Konzentration an den Ionen $Ag^+$ + $Ag^{2+}$ oder $Ag^+$ + $Ag^{2+}$ + $Ag^{3+}$ in der erhaltenen Lösung ein Redoxpotential von mehr als 1000 mV ergibt; und
(D) ein Stabilisierungsmittel, bei dem es sich um eine Säure handelt.

**3.** Zusammensetzung nach Anspruch 2, **dadurch gekennzeichnet, dass** es sich bei den Metallionen um Ionen von $Mo^{VI}$, ein Gemisch von $Ag^+/Ag^{2+}$-Ionen, ein Gemisch von $Mo^{VI}/Ag^+$-Ionen oder um ein Gemisch von $Mo^{VI}/Ag^+/Ag^{2+}$-Ionen handelt.

**4.** Verfahren zur Herstellung einer wässrigen Zusammensetzung nach Anspruch 1 oder 2, wobei das Verfahren **dadurch gekennzeichnet ist, dass** es folgende Stufen umfasst:

(a) Bereitstellen einer wässrigen Lösung einer Quelle L, die Metallionen, die aus folgender Gruppe ausgewählt sind, liefert :

(1) Ionen eines Metalls M, bei denen es sich um die Ionen $Ag^{II}$, $Ag^{III}$, $V^V$, $Nb^V$, $Ta^V$, $Mo^{VI}$, $W^{VI}$, $In^{III}$ oder $Tl^{III}$ handelt,
(2) Kombinationen von $Ag^+$-Ionen und Ionen des Metalls M und
(3) Gemische davon;

wobei die Quelle L, die die Ionen von $Ag^{II}$ oder die Ionen von $Ag^{II}$ + $Ag^{III}$ liefert, so beschaffen ist, dass ausgehend von 100 g Silber (d. h. 157 g $AgNO_3$) und 1 Liter entmineralisiertem Wasser die Konzentration an den Ionen $Ag^+$ + $Ag^{2+}$ oder $Ag^+$ + $Ag^{2+}$ + $Ag^{3+}$ in der erhaltenen Lösung ein Redoxpotential von mehr als 1000 mV ergibt;
(b) Einbringen des Stabilisierungsmittels in diese Lösung;
(c) Einbringen der auf diese Weise erhaltenen Lösung in Wasserstoffperoxid oder Einbringen von Wasserstoffperoxid in die erhaltene Lösung;
(d) Einbringen einer sauren Substanz, bei der es sich um $RCO_3H$, $RCO_2H$ oder um ein $RCO_3H$ + $RCO_2H$-Gemisch handelt, wobei R die vorstehend definierte Bedeutung hat, in die auf diese Weise erhaltene Lösung;
(e) Ruhenlassen der auf diese Weise erhaltenen Lösung bis zur Einstellung des Gleichgewichts

$$H_2O_2 + RCO_2H \Leftrightarrow H_2O + RCO_3H;$$

und
(f) Auffüllen auf 100 Gew.-% mit Wasser.

**5.** Verfahren nach Anspruch 4 zur Herstellung einer Zusammensetzung, die $Ag^{2+}$-Ionen enthält, wobei das Verfahren **dadurch gekennzeichnet ist, dass** es folgende Stufen umfasst:

(1°) Herstellen einer wässrigen Lösung einer Silberverbindung, die als Quelle für $Ag^+$-Ionen dient;
(2°) Oxidieren von mindestens einem Teil der $Ag^+$-Ionen zu $Ag^{2+}$- oder $Ag^{2+}$ + $Ag^{3+}$-Ionen mit Persulfat in der Weise, dass ein Redoxpotential von 1000 mV oder mehr ausgehend von 1 Liter entmineralisiertem Wasser und 157 g $AgNO_3$ erhalten wird, und Filtrieren zur Entfernung von gegebenenfalls vorhandenen unlöslichen Bestandteilen;
(3°) Einbringen des Stabilisierungsmittels in die auf diese Weise erhaltene Lösung;
(4°) Einbringen der auf diese Weise erhaltenen Lösung in Wasserstoffperoxid oder Einbringen von Wasserstoffperoxid in die auf diese Weise erhaltene Lösung;
(5°) Einbringen einer sauren Substanz, bei der es sich um $RCO_3H$, $RCO_2H$ oder um ein $RCO_3H$ + $RCO_2H$-Gemisch handelt, wobei R die vorstehend definierte Bedeutung hat, in die auf diese Weise erhaltene Lösung;
(6°) Ruhenlassen der auf diese Weise erhaltenen Lösung bis zur Einstellung des Gleichgewichts

$$H_2O_2 + RCO_2H \Leftrightarrow H_2O + RCO_3H;$$

und
(7°) Auffüllen auf 100 Gew.-% mit Wasser.

**6.** Zusammensetzung nach Anspruch 1 oder 2, **dadurch gekennzeichnet, dass** sie in Form einer gelierten Zusammensetzung (II) vorliegt, die die wässrige Zusammensetzung nach Anspruch 1 oder 2 und ein anorganisches oder organisches gelbildendes Mittel enthält.

**7.** Zusammensetzung nach Anspruch 6, **dadurch gekennzeichnet, dass** sie folgendes umfasst:

($\alpha$) 50 bis 99 Gew.-% der wässrigen Zusammensetzung (I) nach Anspruch 1 oder 2 und
($\beta$) 50 bis 1 Gew.-% pyrogenes kolloidales Siliciumdioxid mit einer Masseoberfläche von 80 bis 400 $m^2/g$ und einer durchschnittlichen Korngröße von 7 bis 20 nm als gelbildendes Mittel.

**8.** Zusammensetzung nach Anspruch 6, **dadurch gekennzeichnet, dass** sie folgendes umfasst:

($\alpha$) 90 bis 99,7 Gew.-% der wässrigen Zusammensetzung (I) nach Anspruch 1 oder 2 und
($\beta$) 10 bis 0,3 Gew.-% eines organischen Polyacrylmaterials als gelbildendes Mittel.

**9.** Verwendung der gelierten Zusammensetzung nach einem der Ansprüche 6 bis 8, **dadurch gekennzeichnet, dass** man die gelierte Zusammensetzung als keimtötendes Mittel zur Herstellung eines Arzneimittels bereitstellt, das zur Verwendung bei bukkalen Leiden, insbesondere bei Paradontose, aufgrund von Bakterien und/oder Pilzen bestimmt ist.

**10.** Verwendung der gelierten Zusammensetzung nach einem der Ansprüche 6 bis 8, **dadurch gekennzeichnet, dass** man die gelierte Zusammensetzung zur Herstellung eines kosmetischen Produkts für die bukkale Hygiene bereitstellt.

**11.** Zusammensetzung der gelierten Zusammensetzung nach einem der Ansprüche 6 bis 8, **dadurch gekennzeichnet, dass** man die gelierte Zusammensetzung (i) zum Reinigen, Abbeizen und/oder Passivieren einer metallischen Oberfläche oder (ii) zum Reinigen und/oder Abbeizen einer nicht-metallischen Oberfläche bereitstellt.

**12.** Verfahren zur Herstellung der gelierten Zusammensetzung (II) nach Anspruch 6, **dadurch gekennzeichnet, dass** es aus folgenden Stufen besteht: (i) Kontaktieren der Zusammensetzung (I) mit einem gelbildenden Mittel, (ii) 3-bis 20-minütiges Rühren des erhaltenen Gemisches und (iii) Ruhenlassen bis zum Verdicken des erhaltenen

Gemisches.

## Claims

1. An aqueous oxidizing composition (I) which is useful in particular in the field of disinfection, hygiene and decontamination, on the one hand, and in the field of treating surfaces (in particular for cleaning, stripping and/or passivating), on the other hand, said composition, which contains, in water, hydrogen peroxide, an $RCO_2H/RCO_3H$ mixture (in which R is a $C_1$-$C_6$ aliphatic residue containing a linear or branched, saturated or unsaturated hydrocarbon chain) and a stabilizer which is an acid and, where appropriate, $Ag^+$ ions, being **characterized in that** it contains ions of at least one metal M which are $Ag^{II}$, $Ag^{III}$, $V^V$, $Nb^V$, $Ta^V$, $Mo^{VI}$, $W^{VI}$, $Co^{III}$, $In^{III}$ or $Tl^{III}$ ions, the source L, which gives $Ag^{II}$ ions or $Ag^{II}$ + $Ag^{III}$ ions, being such that, starting with 100 g of silver (i.e. 157 g of $AgNO_3$) and 1 liter of demineralized water, the concentration of $Ag^+ + Ag^{2+}$ or $Ag^+ + Ag^{2+} + Ag^{3+}$ ions in the resulting solution gives a redox potential of greater than 1 000 mV.

2. The composition as claimed in claim 1, **characterized in that** it contains, in water:

   (A) $H_2O_2$;
   (B) an $RCO_2H/RCO_3H$ mixture (in which R is a $C_1$-$C_6$ aliphatic residue containing a linear or branched, saturated or unsaturated hydrocarbon chain);
   (C) metal ions from a source L and chosen from the set consisting of:

      (1) ions of a metal M which are $Ag^{II}$, $Ag^{III}$, $V^V$, $Nb^V$, $Ta^V$, $Mo^{VI}$, $W^{VI}$, $Co^{III}$, $In^{III}$ or $Tl^{III}$ ions,
      (2) combinations of $Ag^+$ ions and of ions of said metal M, and
      (3) mixtures thereof, the source L, which gives $Ag^{II}$ ions or $Ag^{II}$ + $Ag^{III}$ ions, being such that, starting with 100 g of silver (i.e. 157 g of $AgNO_3$) and 1 liter of demineralized water, the concentration of $Ag^+ + Ag^{2+}$ or $Ag^+ + Ag^{2+} + Ag^{3+}$ ions in the resulting solution gives a redox potential of greater than 1 000 mV; and

   (D) a stabilizer which is an acid.

3. The composition as claimed in claim 2, **characterized in that** said metal ions are $Mo^{VI}$ ions, a mixture of $Ag^+/Ag^{2+}$ ions, a mixture of $Mo^{VI}/Ag^+$ ions or a mixture of $Mo^{VI}/Ag^+/Ag^{2+}$ ions.

4. A process for preparing an aqueous composition as claimed in claim 1 or 2, said process being **characterized in that** it comprises the steps consisting in

   (a) using an aqueous solution of a source L giving metal ions chosen from the set consisting of

      (1) ions of a metal M which are $Ag^{II}$, $Ag^{III}$, $V^V$, $Nb^V$, $Ta^V$, $Mo^{VI}$, $W^{VI}$, $In^{III}$ or $Tl^{III}$ ions,
      (2) combinations of $Ag^+$ ions and of ions of said metal M, and
      (3) mixtures thereof;
      the source L, which gives $Ag^{II}$ ions or $Ag^{II}$ + $Ag^{III}$ ions, being such that, starting with 100 g of silver (i.e. 157 g of $AgNO_3$) and 1 liter of demineralized water, the concentration of $Ag^+ + Ag^{2+}$ or $Ag^+ + Ag^{2+} + Ag^{3+}$ ions in the resulting solution gives a redox potential of greater than 1 000 mV;

   (b) introducing the stabilizer into said solution:
   (c) introducing said resulting solution thus obtained into aqueous hydrogen peroxide solution or introducing the aqueous hydrogen peroxide solution into said resulting solution;
   (d) introducing into the resulting solution thus obtained an acidic substance which is $RCO_3H$, $RCO_2H$ or a mixture thereof $RCO_3H + RCO_2H$ in which R is defined as indicated above;
   (e) leaving the resulting solution thus obtained to stand until the equilibrium $H_2O_2 + RCO_2H \Leftrightarrow H_2O + RCO_3H$ is established; and
   (f) making up to 100% by weight with water.

5. The process as claimed in claim 4, for preparing a composition which contains $Ag^{2+}$ ions, said process being **characterized in that** it comprises the steps consisting in

   (1°) preparing an aqueous solution of a silver component, which acts as a source of $Ag^+$ ions;

(2°) oxidizing at least some of the $Ag^+$ ions to $Ag^{2+}$ or $Ag^{2+}$ + $Ag^{3+}$ ions using persulfate, so as to obtain a redox potential of greater than or equal to 1 000 mV, starting with 1 liter of demineralized water and 157 g of $AgNO_3$, and filtering to remove any insoluble material which may be present;
(3°) introducing the stabilizer into the resulting solution thus obtained;
(4°) introducing said resulting solution thus obtained into aqueous hydrogen peroxide solution or introducing the aqueous hydrogen peroxide solution into said resulting solution;
(5°) introducing into the resulting solution thus obtained an acidic substance which is $RCO_3H$, $RCO_2H$ or a mixture thereof $RCO_3H + RCO_2H$ in which R is defined as indicated above;
(6°) leaving the resulting solution thus obtained to stand until the equilibrium $H_2O_2 + RCO_2H \Leftrightarrow H_2O + RCO_3H$ is established; and
(7°) making up to 100% by weight with water.

**6.** The composition as claimed in claim 1 or 2, **characterized in that** it is in the form of a gelled composition (II) which comprises the aqueous composition as claimed in claim 1 or 2 and a mineral or organic gelling agent.

**7.** The composition as claimed in claim 6, **characterized in that** it comprises:

(α) 50% to 99% by weight of the aqueous composition (I) as claimed in claim 1 or 2, and
(β) 50% to 1% by weight of pyrogenic colloidal silica with an area per unit mass of from 80 to 400 $m^2/g$ and a mean particle size of from 7 to 20 nm, as gelling agent.

**8.** The composition as claimed in claim 6, **characterized in that** it comprises:

(α) 90% to 99.7% by weight of the aqueous composition (I) as claimed in claim 1 or 2, and
(β) 10% to 0.3% by weight of a polyacrylic organic material as gelling agent.

**9.** The use of the gelled composition as claimed in any one of claims 6 to 8, **characterized in that** said gelled composition is used as a germicidal product for preparing a medicinal product intended for use with regard to oral conditions, in particular those of the periodontium, caused by bacteria and/or fungi.

**10.** The use of the gelled composition as claimed in any one of claims 6 to 8, **characterized in that** said gelled composition is used to prepare a cosmetic product for oral hygiene.

**11.** The use of the gelled composition as claimed in any one of claims 6 to 8, **characterized in that** said gelled composition (i) is used for cleaning, stripping and/or passivating a metal surface, or (ii) for cleaning and/or stripping a nonmetal surface.

**12.** A process for preparing the gelled composition (II) as claimed in claim 6, said process being **characterized in that** it consists (i) in placing said composition (I) in contact with a gelling agent, (ii) in stirring the resulting mixture for 3 to 20 minutes, and (iii) in leaving the resulting mixture to stand in order for it to set to a gel.